# EUROPÄISCHE PATENTSCHRIFT

(11) **EP 4 297 678 B1**
(45) Veröffentlichungstag und Bekanntmachung des Hinweises auf die Patenterteilung: **19.11.2025**
(21) Anmeldenummer: 22707422.6
(22) Anmeldetag: 21.02.2022
(51) Int. Cl.: A61B 18/14, A61M 25/00, A61N 1/04

(54) **GRUNDELEMENT FÜR EINE ELEKTROMEDIZINISCHE VORRICHTUNG UND ELEKTROMEDIZINISCHE VORRICHTUNG**
MAIN ELEMENT FOR AN ELECTROMEDICAL DEVICE, AND ELECTROMEDICAL DEVICE
ÉLÉMENT DE BASE POUR DISPOSITIF ÉLECTROMÉDICAL ET DISPOSITIF ÉLECTROMÉDICAL

(30) Priorität: 23.02.2021 DE 102021104313
(43) Veröffentlichungstag der Anmeldung: 03.01.2024
(73) Patentinhaber: Osypka AG, 79618 Rheinfelden-Baden (DE)
(72) Erfinder: BURG, Benjamin, 79618 Rheinfelden (DE); GÖTTSCHE, Thorsten, 79618 Rheinfelden (DE)
(74) Vertreter: Mertzlufft-Paufler, Cornelius
(86) Internationale Anmeldenummer: PCT/EP2022/054270
(87) Internationale Veröffentlichungsnummer: WO 2022/179985

(56) Entgegenhaltungen:
- DE-A1- 102017 111 280
- US-A1- 2009 240 314
- US-B2- 8 742 814
- MAXIM INTEGRATED: "High-Sensitive Pulse Oximeter and Heart-Rate Sensor for Wearable Health", 20 June 2020 (2020-06-20), XP093204611, Retrieved from the Internet <URL:https://www.analog.com/media/en/technical-documentation/data-sheets/max30101.pdf> [retrieved on 20240912]

## Beschreibung

Die Erfindung betrifft ein Grundelement für eine elektromedizinische Vorrichtung und eine elektromedizinische Vorrichtung, nämlich eine elektromedizinische Elektrode oder einen elektromedizinischen Katheter.

Die US 2009/240 314 A1 offenbart ein implantierbares Elektrodenleitungssystem, das Folgendes umfasst:
eine Reihe von übereinander gestapelten Zwischenlagen,
eine Reihe von ersten Komponenten, wobei sich eine erste Komponente von einer der Zwischenlagen und eine andere erste Komponente von einer anderen der Zwischenlagen aus erstreckt,
eine Reihe von zweiten Bauteilen, und eine Reihe von Verbindern, die die Reihe der ersten Komponenten mit der Reihe der zweiten Komponenten verbinden, wobei die Unterlegscheiben die ersten Komponenten in einer dreidimensionalen Anordnung positionieren.

Aus dem Datenblatt "High-Sensivity Pulse Oximeter and Hear-Rate Sensor for Wearable Health, MAX30101" sind ein Pulsoximeter und ein Herzfrequenzsensor für tragbare Geräte, Fitnessgeräte, Smartphones und Tablets vorbekannt.

Elektromedizinische Vorrichtungen, wie beispielsweise elektromedizinische Elektroden oder auch elektromedizinische Katheter, werden dazu verwendet, um elektromedizinische Untersuchungen und/oder Anwendungen im Inneren des Körpers eines Patienten durchzuführen. Je nach Ausführungsform der elektromedizinischen Vorrichtung werden hierbei elektromedizinische Signale aus dem Körper aufgenommen und an eine an eine Empfangseinheit mit einer Auswerteeinheit der elektromedizinischen Vorrichtung übertragen oder auch Impulse von einem medizinischem Impulsgeber, beispielsweise einem Schrittmacher, auf ein Zielgewebe im Körper des Patienten abgegeben.

Aufgabe der Erfindung ist es, die Gebrauchseigenschaften elektromedizinischer Vorrichtungen, wie beispielsweise elektromedizinischer Elektroden oder auch elektromedizinischer Katheter, zu verbessern.

Zur Lösung der Aufgabe wird zunächst ein Grundelement für eine elektromedizinische Vorrichtung, nämlich für eine implantierbare elektromedizinische Elektrode und/oder einen elektromedizinischen Katheter, vorgeschlagen, das die Mittel und Merkmale des unabhängigen auf ein derartiges Grundelement gerichteten Anspruchs aufweist. Zur Lösung der Aufgabe wird somit bei einem solchen Grundelement vorgeschlagen, dass das Grundelement ein Trägerelement aufweist, an dem zumindest ein Sensormodul und/oder zumindest eine elektromedizinisches Stimulationsmittel, zumindest ein mit dem Sensormodul und/oder mit dem Stimulationsmittel verbundenes Verarbeitungsmodul und zumindest eine Datenleitung angeordnet sind, an die das Verarbeitungsmodul angeschlossen ist.

Als zumindest ein Stimulationsmittel kann beispielsweise zumindest ein elektromedizinisches Stimulationsmittel, insbesondere ein Stimulationspol und/oder ein Ablationspol, zumindest ein fotomedizinisches Stimulationsmittel, insbesondere eine Stimulationsdiode, und/oder zumindest ein biochemisches Stimulationsmittel vorgesehen sein.

Die Datenleitung kann mit einer digital-elektrischen Übertragungsschnittstelle des Grundelements oder einer mit dem Grundelement ausgestatteten Vorrichtung verbunden sein.

Das Sensormodul kann so ausgebildet sein, dass das Grundelement als Grundelement einer Sensing- oder Mapping-Elektrode verwendet werden kann.

Mit dem erfindungsgemäßen Grundelement und den daran angeordneten Funktionseinheiten, nämlich dem zumindest einen Sensormodul, dem zumindest einen Verarbeitungsmodul und der Datenleitung, ist ein modularer Aufbau einer elektromedizinischen Vorrichtung möglich. Je nach Anwendungsfall können mehrere der zuvor genannten Grundelemente miteinander verbunden und so eine maßgeschneiderte elektromedizinische Vorrichtung bereitgestellt werden.

Eine weitere Besonderheit des erfindungsgemäßen Grundelements besteht in dem Verarbeitungsmodul. Mit dem Verarbeitungsmodul ist es möglich, noch an Bord des Grundelements von dem Sensormodul erzeugte Sensorsignale in verarbeitete Datensignale umzuwandeln und über die Datenleitung von dem Grundelement an eine zumindest mittelbar mit dem Grundelement verbundenen Empfangseinheit, beispielsweise mit einer Auswerteeinheit, zu übertragen. Durch das Verarbeitungsmodul ist das Grundelement mit Rechenleistung ausgestattet, die eine Verarbeitung der Sensorsignale ermöglich. Dies kann zu einer Entlastung einer angeschlossenen Empfangseinheit beitragen.

Die Datenleitung kann einen elektrischen Leiter und/oder einen optischen Leiter umfassen. Bei Verwendung eines optischen Leiters kann gegebenenfalls auch eine Energieversorgung des Sensormoduls, des Verarbeitungsmoduls und/oder des zumindest einen Stimulationsmittels optisch durch Licht erfolgen.

Bei der Verarbeitung der von dem zumindest einem Sensormodul erzeugten Sensorsignale kann das Verarbeitungsmodul bereits eine Auswertung und/oder Interpretation der Sensorsignale und eine Umwandlung der Sensorsignale in eine zu überwachende Auswertegröße vornehmen. Auf diese Weise ist es möglich, mit Hilfe des Verarbeitungsmoduls aus einer mit dem Sensormodul überwachten physikalischen Größe auf eine interessierende Auswertegröße rückzuschließen. Bei einem konkreten Ausführungsbeispiel, das weiter unten noch näher erläutert wird, kann das Sensormodul beispielsweise einen Dehnungsmessstreifen und/oder einen Kraftsensor und/oder einen Beschleunigungssensor aufweisen.

Aus der Belastung, die beispielsweise mit einem Dehnungsmessstreifen ermittelt werden kann, lässt sich unter Berücksichtigung weiterer Parameter, beispielsweise einer Steifigkeit und/oder Geometrie des Trägerelements des Grundelements, auf eine Verformung und/oder räumliche Orientierung des Grundelements rückschließen. Genau diese Interpretation der mit dem Sensormodul erfassten physikalischen Größe kann dabei mit Hilfe der Verarbeitungseinheit an Bord des Grundelements vorgenommen werden.

Das Verarbeitungsmodul kann also dazu eingerichtet sein, ein mit dem Sensormodul erzeugtes Sensorsignal auf dem Grundelement zu verarbeiten.

Bei einer Ausführungsform des Grundelements, das einen modularen Aufbau einer elektromedizinischen Vorrichtung, wie beispielsweise einer elektromedizinischen Elektrode oder eines elektromedizinischen Katheters, begünstigt, ist vorgesehen, dass an dem Trägerelement des Grundelements zumindest eine Anschlussschnittstelle zum Anschluss des Grundelements an ein anderes und/oder baugleiches Grundelement, wie mit einem der auf ein Grundelement gerichteten Ansprüche beansprucht ist, ausgebildet ist. Auf diese Weise ist es möglich, die Datenleitungen der Grundelemente über die Anschlussschnittstellen der Grundelemente miteinander zu verbinden. Miteinander verbundene Grundelemente können somit über eine gemeinsame Datenleitung mit einer Empfangseinheit, beispielsweise einer Auswerteeinheit, verbunden werden. Eine separate Verkabelung zum Anschluss der Grundelemente kann hierbei entbehrlich sein. Mehrere miteinander verbundene Grundelemente können untereinander und mit einer angeschlossenen Empfangseinheit mittels eines Bussystems kommunizieren

Um aus mehreren Grundelementen eine elektromedizinische Vorrichtung herzustellen, kann es ferner zweckmäßig sein, wenn an dem Trägerelement des Grundelements zumindest eine Verbindungsschnittstelle zur Verbindung des Grundelements mit einem anderen und/oder baugleichen Grundelement nach einem der auf ein solches gerichteten Ansprüche ausgebildet ist. Die Verbindungsschnittstelle kann insbesondere zur mechanischen Verbindung zweier Grundelemente genutzt werden.

Bei einer Ausführungsform des Grundelements ist vorgesehen, dass eine Anschlussschnittstelle und/oder eine Verbindungsschnittstelle von einem Lötpad an dem Grundelement gebildet ist. Auf diese Weise ist es möglich, zwei miteinander zu verbindende Grundelemente einer elektromedizinischen Vorrichtung über die als Lötpad ausgebildeten Verbindungsschnittstellen und/oder Anschlussschnittstellen sowohl mechanisch als auch signaltechnisch miteinander zu verbinden. Als Anschlussschnittstelle kann beispielsweise die zuvor bereits erwähnte digital-elektrische Übertragungsschnittstelle dienen.

Bei einer Ausführungsform des Grundelements umfasst ein Verarbeitungsmodul eine anwendungsspezifische integrierte Schaltung. Die anwendungsspezifische integrierte Schaltung kann ein ASIC sein. Die anwendungsspezifische integrierte Schaltung kann beispielsweise dazu eingerichtet sein, ein von dem Sensormodul erzeugtes Sensorsignal in eine Auswertegröße umzurechnen und/oder die Auswertegröße und/oder das Sensorsignal über die Datenleitung des Grundelements an eine zumindest mittelbar mit dem Grundelement verbindbare Empfangseinheit, insbesondere eine Auswerteeinheit, zu übertragen. Bei einer Ausführungsform des Grundelements ist vorgesehen, dass die anwendungsspezifische integrierte Schaltung dazu eingerichtet ist, ein von einem Dehnungsmessstreifen und/oder Kraftsensor des Sensormoduls erzeugtes Sensorsignal in eine Kraft als Auswertegröße umzurechnen und diese Auswertegröße über die Datenleitung an die bereits zuvor erwähnte Empfangseinheit zu übertragen.

Das Grundelement, insbesondere das zumindest eine Sensormodul des Grundelements, kann zumindest einen Dehnungsmessstreifen und/oder zumindest einen Kraftsensor und/oder zumindest einen Beschleunigungssensor und/oder zumindest einen biochemischen Sensor und/oder zumindest einen Biosensor für biochemische Körperzustände, insbesondere zur Detektion einer Entzündung, und/oder zumindest einen Temperatursensor und/oder zumindest ein Stimulationsmittel aufweisen. Ein derartiges Stimulationsmittel kann zur Abgabe von Stimulationsimpulsen auf ein Zielgewebe eines Patienten, beispielsweise auf Nervengewebe und/oder Muskelgewebe, eingerichtet sein. Als zumindest ein Stimulationsmittel können beispielsweise zumindest eine optische Stimulationsdiode und/oder zumindest ein elektromedizinischer Stimulationspol und/oder ein weiter unten noch näher erläuterter Ablationspol dienen.

Auf diese Weise ist es möglich, dem Grundelement gemäß den unterschiedlichen Sensortypen und/oder gemäß dem Stimulationsmittel, mit denen es versehen sein kann, unterschiedliche Funktionen zuzuweisen. Bei einer weiter unten noch näher erläuterten elektromedizinischen Vorrichtung, die unterschiedliche Grundelemente der zuvor erläuterten Art umfasst, ist es möglich, dass die Grundelemente unterschiedliche Sensoren aufweisen und/oder zumindest einzelne der Grundelemente auch zumindest ein Stimulationsmittel, wie beispielsweise eine optische Stimulationsdiode und/oder einen elektromedizinischen Stimulationspol, aufweisen. Auf diese Weise wird eine elektromedizinische Vorrichtung geschaffen, die mehrere der zuvor genannten Grundelemente umfasst, denen jeweils unterschiedlichen Funktionen zugewiesen sind.

Um mit dem Grundelement eine Sensing- und/oder Mapping-Elektrode herzustellen, kann das Grundelement, insbesondere das zumindest eine Sensormodul, zumindest einen elektromedizinischen Ableitpol aufweisen. Um auch Ablationsbehandlungen vornehmen zu können, ist es zweckmäßig, wenn das Grundelement, insbesondere das zumindest eine Sensormodul, als Stimulationsmittel zumindest einen elektromedizinischen Ablationspol aufweist. Auf diese Weise können das Grundelement und eine daraus hergestellte Vorrichtung zur therapeutischen Beeinflussung eines Gewebes verwendet werden. Wenn das Grundelement zumindest ein elektromedizinisches Stimulationsmittel aufweist, ist das Grundelement zur Herstellung einer elektromedizinischen Stimulationselektrode geeignet.

Besonders vorteilhaft kann es sein, wenn das Grundelement ein Kompensationsmittel, beispielsweise eine Kompensationsschaltung aufweist, das zur Temperaturkompensation bei der Verarbeitung eines Sensorsignals durch das Verarbeitungsmodul eingerichtet ist. Insbesondere bei solchen Sensormodulen, deren Sensorsignalerzeugung abhängig von der Umgebungstemperatur ist, kann es vorteilhaft sein, eine Kompensation, also beispielsweise eine Korrektur, der erzeugten Sensorsignale unter Berücksichtigung der Umgebungstemperatur vorzunehmen, der das Grundelement bei seiner Verwendung ausgesetzt ist. Dies mit dem Ziel, eine möglichst genaue Überwachung der physikalischen Größe mit dem Sensormodul zu gewährleisten und/oder eine zutreffende Verarbeitung der physikalischen Größe in eine Auswertegröße mit dem Verarbeitungsmodul zu begünstigen. In diesem Zusammenhang kann es besonders vorteilhaft sein, wenn das Grundmodul zumindest einen Temperatursensor aufweist, dessen Messwerte bei der zuvor beschriebenen Kompensation berücksichtigt werden können.

Das Grundelement, insbesondere das zumindest eine Sensormodul, kann ferner eine Detektionsvorrichtung, insbesondere eine Detektionsschaltung, zur Detektion z.B. eines Gewebezustandes oder Gewebekontaktes. Zu diesem Zweck kann die Detektionsvorrichtung beispielsweise zur Impedanzmessung eingerichtet sein. Diese Messung ist hilfreich um aus dem untersuchten Gewebe abgeleitete Signale besser interpretieren zu können und/oder eine Abgabe von Stimulationsimpulsen auf das Gewebe, vorzugsweise automatisch, auszulösen und/oder gegebenenfalls auch zu unterbinden, beispielsweise weil kein ausreichender Kontakt zum Gewebe besteht.

Das Trägerelement des Grundelements kann bei einer bevorzugten Ausführungsform des Grundelements aus Leiterplattenfolie, insbesondere aus einem Zuschnitt aus Leiterplattenfolie, bestehen. Auf diese Weise ist es möglich, die Datenleitung und beispielsweise auch die bereits zuvor erwähnte zumindest eine Anschlussschnittstelle oder gegebenenfalls auch weitere Anschlussschnittstellen, beispielsweise für die das zumindest eine Verarbeitungsmodul und/oder das zumindest eine Sensormodul in die Leiterplattenfolie des Trägerelements zu integrieren oder auf die Leiterplattenfolie zu drucken und/oder auf dieser abzuscheiden.

Bei einer bevorzugten Ausführungsform des Grundelements ist vorgesehen, dass das Trägerelement aus einem ebenen Zuschnitt aus Leiterplattenfolie besteht, der in Gebrauchsstellung des Grundelements zu einem dreidimensionalen, vorzugsweise langgestreckten, Körper entfaltet ist. Auf diese Weise erhält das aus Leiterplattenfolie bestehende Trägerelement des Grundelements eine unter Berücksichtigung des Materialeinsatzes und Eigengewichts hohe Stabilität. In Gebrauchsstellung des Grundelements kann das Trägerelement beispielsweise zu einem dreidimensionalen Körper in Form eines, vorzugsweise geraden, Prismas oder eines Zylinders gefaltet sein.

Das zumindest eine Sensormodul des Grundelements kann bei dem zuvor beschriebenen Grundelement an einer Seite des und/oder in einer Mitte des Trägerelements angeordnet sein, die eine Innenseite des dreidimensionalen Körpers bildet. Auf diese Weise ist das Sensormodul des Grundelements gut geschützt im Inneren des dreidimensionalen Körpers des Grundelements angeordnet. Bei einer Ausführungsform des Grundelements können auch das zumindest eine Verarbeitungsmodul und/oder die zumindest eine Datenleitung an einer Seite des Trägerelements angeordnet sein, die eine Innenseite des dreidimensionalen Körpers bildet. Hierbei ist es möglich, dass das Sensormodul und/oder das zumindest eine Verarbeitungsmodul und/oder die zumindest eine Datenleitung an derselben Innenseite des dreidimensionalen Körpers oder auch an unterschiedlichen Innenseiten des dreidimensionalen Körpers angeordnet sind.

Bei einer Ausführungsform des Grundelements kann die Datenleitung in das Trägerelement integriert sein, insbesondere wenn das Trägerelement aus Leiterplattenfolie besteht.

Bei einer Ausführungsform des Grundelements, bei dem das Trägerelement auf einer Leiterplattenfolie, beispielsweise aus einer PCB-Folie besteht, können die zumindest eine Datenleitung und/oder zumindest Bestandteile des zumindest einen Verarbeitungsmoduls und/oder des zumindest einen Sensormoduls auf die Leiterplattenfolie gedruckt und/oder abgeschieden sind.

Zur Lösung der Aufgabe wird auch eine elektromedizinische Vorrichtung mit den Merkmalen des unabhängigen, auf einer derartigen elektromedizinischen Vorrichtung gerichteten Anspruchs vorgeschlagen. Zur Lösung der Aufgabe wird somit insbesondere eine elektromedizinische Vorrichtung vorgeschlagen, die zumindest ein Grundelement nach einem der auf ein solches gerichteten Ansprüche aufweist. Die elektromedizinische Vorrichtung ist als implantierbare elektromedizinische Elektrode und/oder elektromedizinischer Katheter ausgebildet.

Bei einer Ausführungsform der elektromedizinischen Vorrichtung weist diese nur ein einziges Grundelement auf. Das Grundelement kann sich hierbei von einem distalen bis zu einem proximalen Ende der Vorrichtung erstrecken. Wenn die Vorrichtung als Katheter ausgebildet ist, kann das Grundelement auch ein Anschlusskabel, eine Anschlussleitung und/oder einen Lichtleiter aufweisen, der/die an eine Auswerteeinheit und/oder Steuereinheit der Vorrichtung angeschlossen sein kann/können.

Bei einer Ausführungsform der elektromedizinischen Vorrichtung ist vorgesehen, dass diese zumindest zwei Grundelemente nach jeweils einem der auf ein solches gerichteten Ansprüche aufweist. Dabei können die Verarbeitungsmodule der Grundelemente an eine gemeinsame Datenleitung der elektromedizinischen Vorrichtung angeschlossen sein. Die gemeinsame Datenleitung der elektromedizinischen Vorrichtung kann hierbei zumindest abschnittsweise aus den Datenleitungen der miteinander verbundenen Grundelemente der elektromedizinischen Vorrichtung zusammengesetzt sein oder zumindest abschnittsweise aus diesen Datenleitungen bestehen.

Um der elektromedizinischen Vorrichtung eine gute Stabilität zu verleihen, kann es zweckmäßig sein, dass sich zwei miteinander verbundene Grundelemente zumindest teilweise gegenseitig überlappen.

Verarbeitungsmodule von Grundelementen der elektromedizinischen Vorrichtung können Teil eines Datenbussystems der elektromedizinischen Vorrichtung sein. Hierbei ist es möglich, dass die Verarbeitungsmodule der Grundelemente an eine sich über die Grundelemente der elektromedizinischen Vorrichtung erstreckende, gemeinsame Datenleitung angeschlossen sind. Auf diese Weise kann die Anzahl von Datenleitungen minimiert werden.

Bei einer Ausführungsform der elektromedizinischen Vorrichtung ist vorgesehen, dass diese eine Steuereinheit und/oder eine Empfangseinheit, insbesondere mit einer Auswerteeinheit, aufweist, an die ihr zumindest ein Grundelement zumindest mittelbar angeschlossen ist. Die mit dem Verarbeitungsmodul des zumindest einen Grundelements verarbeiteten Sensorsignale des Sensormoduls können dann an die Empfangseinheit der elektromedizinischen Vorrichtung zur weiteren Verarbeitung und/oder Auswertung übertragen werden.

Ferner ist es möglich, dass die elektromedizinische Vorrichtung zumindest einen medizinischen Impulsgeber aufweist. Der Impulsgeber kann beispielsweise ein elektromedizinischer und/oder fotomedizinischen Impulsgeber sein. Auf diese Weise können elektromedizinische und/oder fotomedizinische und/oder biochemische Impulse an zumindest ein entsprechendes Stimulationsmittel der elektromedizinischen Vorrichtung, das an einem Grundelement der elektromedizinischen Vorrichtung angeordnet oder ausgebildet sein kann, übertragen und von dort auf ein Zielgewebe eines Patienten abgegeben werden. So kann aus den zuvor erwähnten Grundelementen auch eine elektromedizinische Vorrichtung in Form einer Stimulationselektrode, Schrittmacherelektrode und/oder eines solchen Katheters erzeugt werden.

Die elektromedizinische Vorrichtung kann ferner eine Hülle aufweisen, innerhalb der das zumindest eine Grundelement geschützt angeordnet ist. Als Hülle kann beispielsweise ein Hüllschlauch vorgesehen sein. Vorzugsweise sind sämtliche Grundelemente der elektromedizinischen Vorrichtung in einer gemeinsamen Hülle, insbesondere in einem gemeinsamen Hüllschlauch angeordnet.

Bei einer bevorzugten Ausführungsform ist die elektromedizinische Vorrichtung als elektromedizinische Elektrode, insbesondere als Sensing-Elektrode, Mapping-Elektrode und/oder Ablationselektrode ausgebildet. Dabei kann die Vorrichtung als implantierbare elektromedizinische Elektrode ausgebildet sein.

Die elektromedizinische Vorrichtung kann auch als elektromedizinischer Katheter ausgebildet sein.

Die Erfindung wird nachfolgend anhand eines Ausführungsbeispiels näher beschrieben, ist aber nicht auf dieses Ausführungsbeispiel beschränkt. Weitere Ausführungsbeispiele ergeben sich durch Kombination der Merkmale einzelner oder mehrerer Schutzansprüche untereinander und/oder in Kombination einzelner oder mehrerer Merkmale des Ausführungsbeispiels. Es zeigen:
- Fig. 1: eine isometrische Darstellung eines Grundelements, das ein Trägerelement aus Leiterplattenfolie im flachen Ausgangszustand aufweist, sowie an einer Seite des Trägerelements mit einem Verarbeitungsmodul und einem Sensormodul versehen ist,
- Fig. 2: das in Figur 1 gezeigte Grundelement in entfalteter Gebrauchsstellung,
- Fig. 3: insgesamt vier über ihre Anschluss- und Verbindungsschnittstellen miteinander verbundene Grundelemente, wie sie in den Figuren 1 und 2 dargestellt sind,
- Fig. 4: die in Figur 3 im ungefalteten Zustand dargestellten vier Grundelemente in entfalteter Stellung,
- Fig. 5: eine elektromedizinische Vorrichtung in Form einer elektromedizinischen Elektrode mit mehreren Grundelementen, wie sie in den vorherigen Figuren dargestellt sind, wobei die Grundelemente in einem Hüllschlauch der elektromedizinischen Vorrichtung angeordnet sind, sowie
- Fig. 6: eine weitere Ausführungsform einer elektromedizinischen Vorrichtung in Seitenansicht mit einem einzigen Grundelement und einem Korb an ihrem distalen Ende aus Katheterarmen.

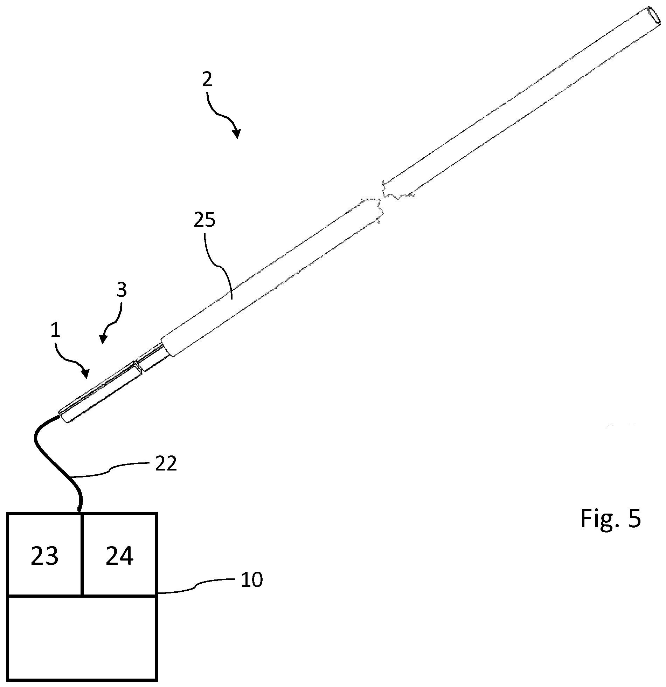

Sämtliche Figuren zeigen im Ganzen mit 1 bezeichnete Grundelemente für eine elektromedizinische Vorrichtung 2. Figur 5 zeigt eine elektromedizinische Vorrichtung 2, die mehrere, miteinander verbundene Grundelemente 1 umfasst und die als elektromedizinische Elektrode, insbesondere als implantierbare elektromedizinische Elektrode, oder auch als elektromedizinischer Katheter verwendet werden kann.

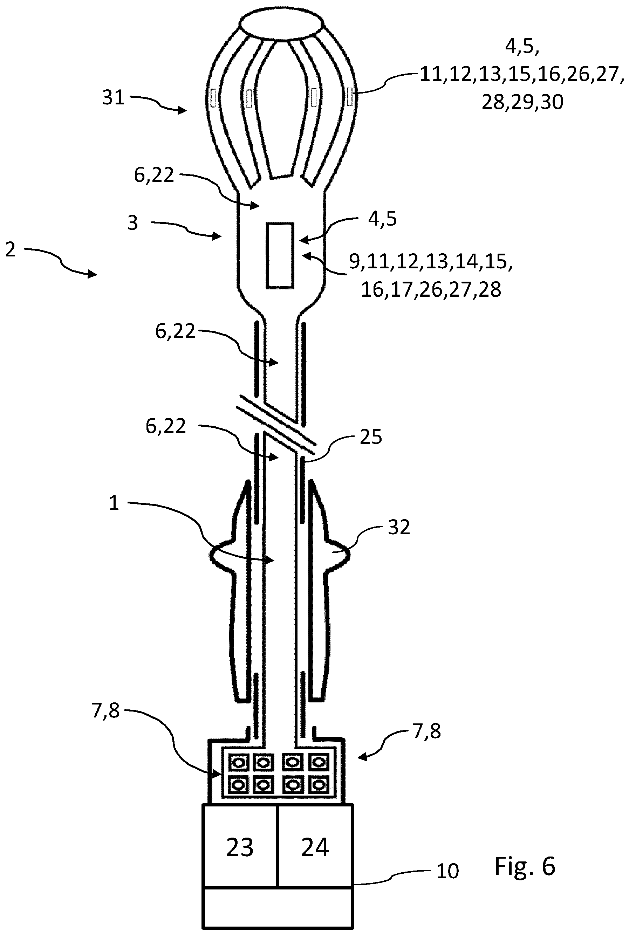

Figur 6 zeigt eine als Basket-Katheter oder Korb-Katheter ausgebildete Vorrichtung 2, die nur ein einziges Grundelement 1 umfasst, das von einem distalen Ende bis zu einem proximalen Ende der Vorrichtung 2 reicht.

Bei der nachfolgenden Beschreibung verschiedener Ausführungsformen der Erfindung erhalten in ihrer Funktion übereinstimmende Elemente auch bei abweichender Gestaltung oder Formgebung übereinstimmende Bezugszahlen.

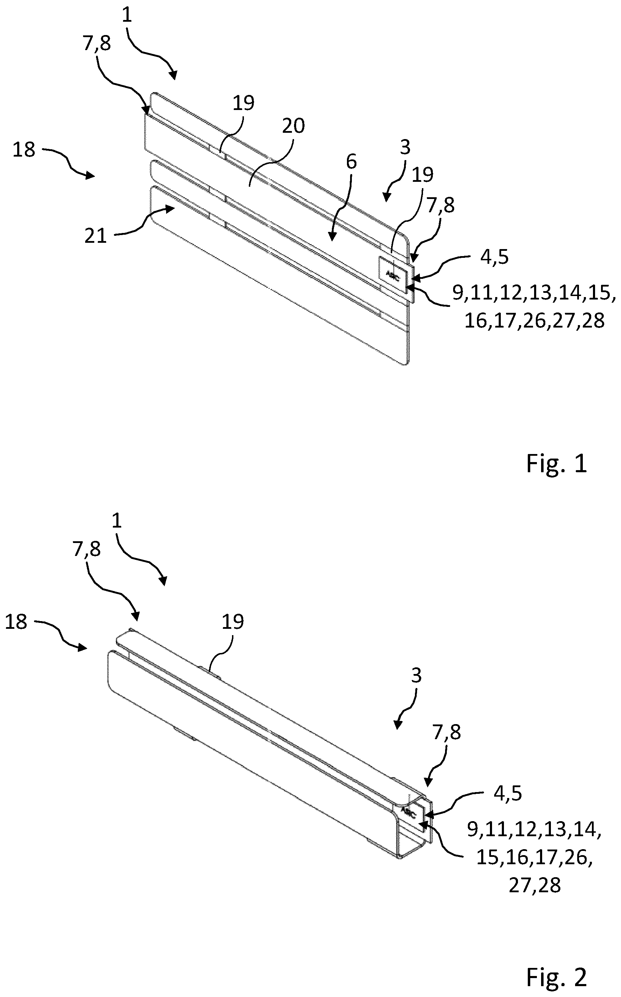

Beispielsweise Figur 1 verdeutlicht, dass das Grundelement 1 ein Trägerelement 3 aufweist. An dem Trägerelement 3 sind ein Sensormodul 4, ein mit dem Sensormodul 4 verbundenes Verarbeitungsmodul 5 und zumindest eine Datenleitung 6 angeordnet, an die das Verarbeitungsmodul 5 angeschlossen ist. Die Datenleitung 6 kann durch einen elektrischen und/oder optischen Lichtleiter gebildet sein.

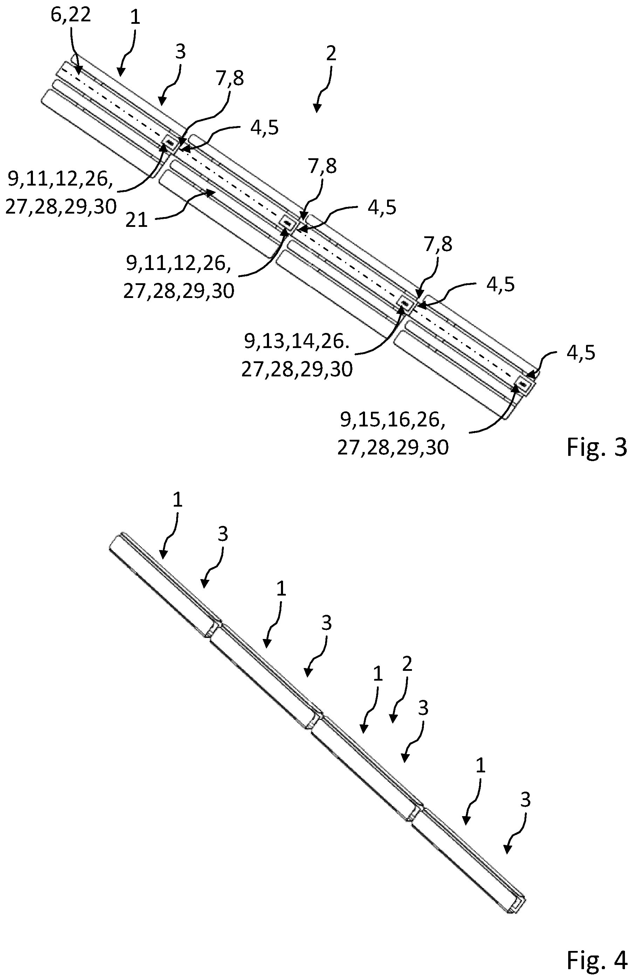

Das Verarbeitungsmodul 5 des Grundelements 1 ist dazu eingerichtet, ein mit dem Sensormodul 4 erzeugtes Sensorsignal noch auf dem Grundelement 1 zu verarbeiten. An dem Trägerelement 3 eines jeden Grundelements 1 sind zwei Anschlussschnittstellen 7 zum Anschluss des Grundelements 1 an andere und/oder baugleiche Grundelemente 1 ausgebildet. Dies ist gut anhand der Figuren 3, 4 und 5 zu erkennen, die jeweils vier miteinander verbundene Grundelemente 1 zeigen.

An den Trägerelementen 3 der Grundelemente 1 sind ferner jeweils zwei Verbindungsschnittstellen 8 zur Verbindung, insbesondere zur mechanischen Verbindung, des jeweiligen Grundelements 1 mit anderen und/oder baugleichen Grundelementen 1 ausgebildet. Bei den in den Figuren gezeigten Grundelementen 1 sind jeweils eine Anschlussschnittstelle 7 und eine Verbindungsschnittstelle 8 in einer Schnittstelle kombiniert.

Die Anschlussschnittstelle 7 und die Verbindungsschnittstelle 8 sind von einem Lötpad an dem Trägerelement 3 des Grundelements 1 gebildet. Mit einem Lötpad ist es möglich, eine signaltechnische Verbindung zwischen den Datenleitungen 6 der Grundelemente 1 einerseits und eine mechanische Verbindung zwischen den Trägerelementen 3 der Grundelemente 1 andererseits herzustellen.

Die Verarbeitungsmodule 5 der gezeigten Vorrichtungen 2 umfassen jeweils zumindest eine anwendungsspezifische integrierte Schaltung 9. Die Schaltungen 9 der Grundelemente 1 sind dazu eingerichtet, ein von dem jeweiligen Sensormodul 4 erzeugtes Sensorsignal in eine Auswertegröße umzuwandeln und die Auswertegröße und/oder bei Bedarf auch das Sensorsignal des Sensormoduls 4 über die Datenleitung 6 an eine zumindest mittelbar mit dem jeweiligen Grundelement verbindbare und in Gebrauchsstellung verbundene Empfangseinheit 10 zu übertragen.

Je nach Ausführungsform weisen die Grundelemente 1, insbesondere ihre Sensormodule 4, jeweils zumindest einen Dehnungsmessstreifen 11, zumindest einen Kraftsensor 12, zumindest einen biochemischen Sensor 13, zumindest einen Biosensor 14 für biochemische Körperzustände, insbesondere zur Detektion einer Entzündung, und/oder zumindest einen Beschleunigungssensor 30 und/oder zumindest einen Temperatursensor 26 auf. Ferner sind die Vorrichtungen 2 auch mit Ableitpolen 27 ausgestattet, über die elektrische Körpersignale aufgenommen und Sensing- und/oder Mapping-Untersuchungen durchgeführt werden können.

Insbesondere die in Figur 6 gezeigte Ausführungsform der Vorrichtung 2 ist durch die an ihrem Katheter-Korb 31 angeordneten Ableitpole 27 besonders gut zur Durchführung von Sensing- und/oder Mapping-Untersuchungen geeignet.

Eines der Grundelemente 1 der in den Figuren gezeigten Vorrichtungen 2 ist zudem mit zumindest einem Stimulationsmittel 15, 16, 28, beispielsweise einer optischen Stimulationselektrode 15 und auch mit zumindest einem elektromedizinischen Stimulationspol 16 sowie mit zumindest einem Ablationspol 28 ausgestattet. Die zuvor erwähnten Sensoren und auch die zuvor erwähnte Stimulationsdiode 15 beziehungsweise der Stimulationspol 16 können an einer in Gebrauchsstellung der Außenseite des Trägerelements 3 des jeweiligen Grundelements 1 angeordnet sein. Mit dem Ablationspol 28 können Impulse zur Ablation von Gewebe abgegeben werden.

Distale Grundelemente 1 der gezeigten Vorrichtungen 1 sind ferner mit Detektionsvorrichtungen 29 ausgestattet. Die Detektionsvorrichtungen 29 sind in die Sensormodule 4 integriert und zur Detektion eines Gewebezustandes oder Gewebekontaktes beispielsweise durch Impedanzmessung eingerichtet.

Die Grundelemente 1 sind zudem mit einem Kompensationsmittel 17, beispielsweise einer Kompensationsschaltung, ausgestattet, das zur Temperaturkompensation bei der Verarbeitung eines Sensorsignals durch das an dem Trägerelement 3 angeordnete Verarbeitungsmodul 5 des jeweiligen Grundelements 1 eingerichtet ist.

Bei sämtlichen Grundelementen 1, die in den Figuren gezeigt sind, bestehen die Trägerelemente 3 aus Leiterplattenfolie.

Die Figuren 1 und 2 sowie 3 und 4 verdeutlichen, dass die Trägerelemente 3 der Grundelemente 1 jeweils aus einem ebenen Zuschnitt aus Leiterplattenfolie bestehen. Die Figuren 1 und 3 zeigen die Grundelemente 1 mit ihren Trägerelementen 3 noch als flachen Zuschnitt. In Gebrauchsstellung, die in den Figuren 2 und 4 sowie 5 zu erkennen ist, sind die Trägerelemente 3 der Grundelemente 1 jeweils zu einem dreidimensionalen, langgestreckten Körper in Form eines geraden Prismas entfaltet. Genauer gesagt weisen die entfalteten Zuschnitte der Trägerelemente 3 eine gedachte Hüllfläche auf, die der eines geraden Prismas entspricht.

Das Sensormodul 4, das Verarbeitungsmodul 5 und auch die Datenleitung 6 eines jeden Grundelements 1 sind an einer Seite des Trägerelements 3 angeordnet sein, die eine Innenseite 17 des dreidimensionalen Körpers bildet, zu dem das Trägerelement in Gebrauchsstellung des Grundelements 1 entfaltet ist. Wie bereits zuvor erwähnt, können Sensoren oder auch Stimulationsdioden 15 beziehungsweise Stimulationspole 16 der Grundelemente 1 an einer Seite des Trägerelements 3 angeordnet sein, die im gefalteten Zustand des Trägerelements 3 eine Außenseite des Trägerelements 3 bildet.

Figur 1 zeigt deutlich, dass das Trägerelement 3 insgesamt vier miteinander verbundene Streifen 18, 20 aus Leiterplattenfolie umfasst. Die Streifen 18, 20 sind über Verbindungsstege 19 miteinander verbunden. Die Verbindungsstege 19 fungieren dabei auch als Filmscharniere, die ein Entfalten des ebenen Zuschnitts des Trägerelements 3 aus der in Figur 1 gezeigten Stellung in die in Figur 2 gezeigte Stellung ermöglichen. Erwähnenswert ist, dass der Streifen 20, an dem das Verarbeitungsmodul 5 mit der anwendungsspezifisch integrierten Schaltung 9 angeordnet ist, länger als die übrigen drei Streifen 18 des Trägerelements 3 ist. Die Darstellung der insgesamt vier miteinander verbundenen Trägerelemente 3 gemäß Figur 3 macht deutlich, dass eine Verbindung der Grundelemente 1 an diesem längsten Streifen 20 der Trägerelemente 3 vorgenommen wird. An den längsten Streifen 20 sind auch die Anschlussschnittstellen 7 und die Verbindungsschnittstellen 8 der Grundelemente 1 angeordnet.

Hierbei ist zu erwähnen, dass an beiden freien Enden eines jeden längsten Streifens 20 des jeweiligen Trägerelements 3 jeweils eine Anschlussschnittstelle 7 und eine Verbindungsschnittstelle 8 ausgebildet sind. Die Datenleitung 6 wie auch die Anschlussschnittstellen 7 und die Verbindungsschnittstellen 8 der Trägerelemente 3 können beispielsweise auf die Trägerelemente 3 aufgedruckt und/oder abgeschieden sein. Gleiches gilt für Anschlussschnittstellen und/oder Bestandteile von Schaltungen der Sensormodule 4, der Verarbeitungsmodule 5 oder auch der Kompensationsmittel 17.

Figur 5 zeigt eine elektromedizinische Vorrichtung 2 in Form einer elektromedizinischen Elektrode beziehungsweise in Form eines elektromedizinischen Katheters. Die elektromedizinische Vorrichtung 2 umfasst mehrere Grundelemente 1, wie sie in den Figuren 1 bis 4 dargestellt sind.

Figur 6 zeigt eine elektromedizinische Vorrichtung 2 in Form einer elektromedizinischen Elektrode beziehungsweise in Form eines elektromedizinischen Katheters. Die elektromedizinische Vorrichtung 2 umfasst ein einziges, durchgängiges Grundelement 1, das von einem distalen zu einem proximalen Ende der Vorrichtung 2 reicht und im distalen Endbereich mehrere Arme aufweist, die zu einem Katheterkorb 31 aufgespannt sind. Einige Sensormodule 4 und insbesondere die Ableitpole 27 und Ablationspole 28 sind an den Armen des Katheterkorbs 31 angeordnet. Der Katheterkorb 31 bildet eine atraumatische Spitze der Vorrichtung 2. Durch den Katheterkorb 31 ist die Vorrichtung 2 gut zum Sensing und/oder Mapping geeignet, also zur Erstellung von Reizerregungs-Karten eines untersuchten Gewebes. Die in Figur 6 abgebildete und als Katheter ausgebildete Vorrichtung 2 weist einen Griff 32 auf, mit sie sich bedienen und im Zielgewebe positionieren lässt.

Die Verarbeitungsmodule 5 der Grundelemente 1 der elektromedizinischen Vorrichtung 2 sind an eine gemeinsame Datenleitung 22 der elektromedizinischen Vorrichtung angeschlossen. Die gemeinsame Datenleitung 22 der elektromedizinischen Vorrichtung 2 wird zumindest abschnittsweise von den Datenleitungen 6 der Grundelemente 1 gebildet.

Die Figuren 3 und 4 deuten an, dass sich zwei miteinander verbundene Grundelemente 1 der elektromedizinischen Vorrichtung 2 zumindest teilweise gegenseitig überlappen. Die Überlappung findet dabei an freien Enden der längsten Streifen 20 der Trägerelemente 3 der Grundelemente 1 statt.

Die Verarbeitungsmodule 5 der Grundelemente 1 der elektromedizinischen Vorrichtungen 2 sind Teil eines Datenbussystems der elektromedizinischen Vorrichtung 2. Sämtliche Verarbeitungsmodule 5 sind an die gemeinsame Datenleitung 22 der elektromedizinischen Vorrichtung 2 angeschlossen.

Die elektromedizinische Vorrichtung 2 ist mit einer Empfangseinheit 10, die eine Auswerteeinheit 23 und einen medizinischen Impulsgeber 24 umfasst, ausgestattet, an die die Grundelemente 1 der elektromedizinischen Vorrichtung 2 über die gemeinsame Datenleitung 22 angeschlossen sind.

Die elektromedizinische Vorrichtung 2 gemäß Figur 5 weist ferner eine Hülle 25, nämlich einen Hüllschlauch, auf, innerhalb der die Grundelemente 1 geschützt angeordnet sind.

Die Erfindung befasst sich mit technischen Verbesserungen auf dem Gebiet der elektromedizinischen Vorrichtungen, insbesondere der elektromedizinischen Elektroden und/oder elektromedizinischen Katheter. Als Verbesserung wird u.a. ein Grundelement 1 für eine elektromedizinische Vorrichtung 2 vorgeschlagen, das an einem Trägerelement 3 zumindest ein Sensormodul 4, zumindest ein Verarbeitungsmodul 5 und zumindest eine Datenleitung 6 aufweist, an die das zumindest eine Verarbeitungsmodul 5 des Grundelements 1 angeschlossen ist. Aus zumindest einem Grundelement 1 oder vorzugsweise aus mehreren Grundelementen 1 kann dann eine elektromedizinische Vorrichtung 2 bedarfsgerecht konfiguriert werden.

### Bezugszeichenliste

- 1: Grundelement
- 2: elektromedizinische Vorrichtung
- 3: Trägerelement
- 4: Sensormodul
- 5: Verarbeitungsmodul
- 6: Datenleitung
- 7: Anschlussschnittstelle
- 8: Verbindungsschnittstelle
- 9: Schaltung
- 10: Empfangseinheit
- 11: Dehnungsmessstreifen
- 12: Kraftsensor
- 13: biochemischer Sensor
- 14: Biosensor
- 15: Stimulationsdiode
- 16: Stimulationspol
- 17: Kompensationsmittel
- 18: Streifen
- 19: Verbindungsstege
- 20: längster Streifen von 3
- 21: Innenseite
- 22: gemeinsame Datenleitung von 2
- 23: Auswerteeinheit
- 24: Impulsgeber
- 25: Hülle
- 26: Temperatursensor
- 27: Ableitpol
- 28: Ablationspol
- 29: Detektionsvorrichtung
- 30: Beschleunigungssensor
- 31: Katheterkorb
- 32: Griff

## Patentansprüche

1. Grundelement (1) für eine elektromedizinische Vorrichtung (2), nämlich für eine implantierbare elektromedizinische Elektrode und/oder einen elektromedizinischen Katheter, wobei das Grundelement (1) ein Trägerelement (3) aufweist, an dem zumindest ein Sensormodul (4) und/oder zumindest ein Stimulationsmittel (15,16,28), zumindest ein mit dem Sensormodul (4) und/oder mit dem Stimulationsmittel (15,16,28) verbundenes Verarbeitungsmodul (5) und zumindest eine Datenleitung (6) angeordnet sind, an die das Verarbeitungsmodul (5) angeschlossen ist.

2. Grundelement (1) nach dem vorherigen Anspruch, wobei das Verarbeitungsmodul (5) dazu eingerichtet ist, ein mit dem Sensormodul (4) erzeugtes Sensorsignal auf dem Grundelement (1) zu verarbeiten.

3. Grundelement (1) nach einem der vorherigen Ansprüche, wobei an dem Trägerelement (3) zumindest eine, insbesondere zwei, Anschlussschnittstelle(n) (7) zum Anschluss des Grundelements (1) an ein anderes und/oder baugleiches Grundelement (1) nach einem der vorherigen Ansprüche ausgebildet ist, und/oder wobei an dem Trägerelement (3) zumindest eine, insbesondere zwei, Verbindungsschnittstelle(n) (8) zur Verbindung des Grundelements (1) mit einem anderen und/oder baugleichen Grundelement (1) nach einem der vorherigen Ansprüche ausgebildet ist.

4. Grundelement (1) nach dem vorherigen Anspruch, wobei die Anschlussschnittstelle (7) und/oder die Verbindungsschnittstelle von einem Lötpad an dem Trägerelement (3) gebildet ist.

5. Grundelement (1) nach einem der vorherigen Ansprüche, wobei zumindest ein Verarbeitungsmodul (5) eine anwendungsspezifische integrierte Schaltung (9) umfasst, insbesondere die dazu eingerichtet ist, ein von dem Sensormodul (4), insbesondere von einem oder dem Dehnungsmessstreifen des Sensormoduls (4), erzeugtes Sensorsignal in eine Auswertegröße, insbesondere in eine Kraft, umzurechnen und/oder die Auswertegröße und/oder das Sensorsignal über die Datenleitung (6) an eine zumindest mittelbar mit dem Grundelement (1) verbindbare Empfangseinheit (10) zu übertragen.

6. Grundelement (1) nach einem der vorherigen Ansprüche, wobei das Grundelement (1), insbesondere das zumindest eine Sensormodul (4), zumindest einen Dehnungsmessstreifen (11) und/oder zumindest einen Kraftsensor (12) und/oder zumindest einen Beschleunigungssensor (30) und/oder zumindest einen biochemischen Sensor (13) und/oder zumindest einen Biosensor (14) für biochemische Körperzustände, insbesondere zur Detektion einer Entzündung, und/oder zumindest einen Temperatursensor (26) und/oder zumindest einen elektrischen Ableitpol (27) und/oder als zumindest ein Stimulationsmittel zumindest eine optische Stimulationsdiode (15) und/oder zumindest einen elektromedizinischen Stimulationspol (16) und/oder zumindest einen elektromedizinischen Ablationspol (28) aufweist.

7. Grundelement (1) nach einem der vorherigen Ansprüche, wobei das Grundelement (1) ein Kompensationsmittel (17), insbesondere eine Kompensationsschaltung, aufweist, das zur Temperaturkompensation bei der Verarbeitung eines Sensorsignals durch das Verarbeitungsmodul (5) eingerichtet ist.

8. Grundelement (1) nach einem der vorherigen Ansprüche, wobei das Grundelement (1), insbesondere das zumindest eine Sensormodul (4), eine Detektionsvorrichtung (29), insbesondere eine Detektionsschaltung, zur Detektion eines Gewebezustandes oder Gewebekontaktes aufweist, insbesondere wobei die Detektionsvorrichtung (29) zur Impedanzmessung eingerichtet ist.

9. Grundelement (1) nach einem der vorherigen Ansprüche, wobei das Trägerelement (3) aus Leiterplattenfolie, insbesondere aus einem Zuschnitt aus Leiterplattenfolie, besteht.

10. Grundelement (1) nach einem der vorherigen Ansprüche, wobei das Trägerelement (3) aus einem ebenen Zuschnitt aus Leiterplattenfolie besteht, insbesondere der in Gebrauchsstellung des Grundelements (1) zu einem dreidimensionalen, vorzugsweise langgestreckten, Körper, insbesondere zu einem, vorzugsweise geraden, Prisma oder einem Zylinder, entfaltet ist.

11. Grundelement (1) nach dem vorherigen Anspruch, wobei das zumindest eine Sensormodul (4) und/oder das zumindest eine Verarbeitungsmodul (5) und/oder die zumindest eine Datenleitung (6) an einer Seite des Trägerelements (3) angeordnet sind, die eine Innenseite (17) des dreidimensionalen Körpers bildet.

12. Elektromedizinische Vorrichtung (2), nämlich implantierbare elektromedizinische Elektrode und/oder elektromedizinischer Katheter, mit zumindest einem Grundelement (1) nach einem der vorherigen Ansprüche.

13. Elektromedizinische Vorrichtung (2) nach dem vorherigen Anspruch mit zumindest zwei Grundelementen (1) nach einem der vorherigen Ansprüche, deren Verarbeitungsmodule (5) an eine gemeinsame Datenleitung (22) der elektromedizinischen Vorrichtung (2) angeschlossen sind, insbesondere wobei die Datenleitung (6,22) eine elektrische und/oder optische Datenleitung, insbesondere ein Lichtleiter, ist.

14. Elektromedizinische Vorrichtung (2) nach einem der Ansprüche 12 oder 13, wobei sich zwei miteinander verbundene Grundelemente (1) zumindest teilweise gegenseitig überlappen.

15. Elektromedizinische Vorrichtung (2) nach einem der Ansprüche 12 bis 14, wobei Verarbeitungsmodule (5) von Grundelementen (1) der elektromedizinischen Vorrichtung Teil eines Datenbussystems der elektromedizinischen Vorrichtung (2) sind.

16. Elektromedizinische Vorrichtung (2) nach einem der Ansprüche 12 bis 15, mit einer Empfangseinheit (10), insbesondere mit einer Auswerteeinheit (23), an die das zumindest eine Grundelement (1) zumindest mittelbar angeschlossen ist, und/oder mit einem medizinischen Impulsgeber (24), an die das zumindest eine Grundelement (1) zumindest mittelbar angeschlossen ist.

17. Elektromedizinische Vorrichtung (2) nach einem der Ansprüche 12 bis 16 mit einer Hülle (25), insbesondere mit einem Hüllschlauch, innerhalb der/dessen das zumindest eine Grundelement (1) angeordnet ist.

18. Elektromedizinische Vorrichtung (2) nach einem der Ansprüche 12 bis 17, wobei die elektromedizinische Vorrichtung (2) als implantierbare elektromedizinische Elektrode, nämlich als Mapping-Elektrode und/oder Sensing-Elektrode und/oder Ablations-Elektrode, und/oder als elektromedizinischer Katheter ausgebildet ist.

## Claims

1. Base element (1) for an electromedical device (2), specifically for an implantable electromedical electrode and/or an electromedical catheter, wherein the base element (1) comprises a carrier element (3) on which at least one sensor module (4) and/or at least one stimulation means (15, 16, 28), at least one processing module (5) connected to the sensor module (4) and/or to the stimulation means (15, 16, 28), and at least one data line (6) are arranged, to which the processing module (5) is connected.

2. Base element (1) according to the preceding claim, wherein the processing module (5) is designed to process a sensor signal generated by the sensor module (4) on the base element (1).

3. Base element (1) according to one of the preceding claims, wherein at least one, in particular two, connection interfaces (7) for connecting the base element (1) to another and/or structurally identical base element (1) according to one of the preceding claims are formed on the carrier element (3), and/or wherein at least one, in particular two, interconnect interfaces (8) are formed on the carrier element (3) for connecting the base element (1) to another and/or identically constructed base element (1) according to one of the preceding claims.

4. Base element (1) according to the preceding claim, wherein the connection interface (7) and/or the interconnect interface is formed by a solder pad on the carrier element (3).

5. Base element (1) according to one of the preceding claims, wherein at least one processing module (5) comprises an application-specific integrated circuit (9), which in particular is designed to convert a sensor signal generated by the sensor module (4), in particular from one or the strain gauges of the sensor module (4), into an evaluation variable, in particular into a force, and/or to transmit the evaluation variable and/or the sensor signal via the data line (6) to a receiving unit (10) that can be connected at least indirectly to the base element (1).

6. Base element (1) according to one of the preceding claims, wherein the base element (1), in particular the at least one sensor module (4), comprises at least one strain gauge (11) and/or at least one force sensor (12) and/or at least one acceleration sensor (30) and/or at least one biochemical sensor (13) and/or at least one biosensor (14) for biochemical body conditions, in particular for detecting inflammation, and/or at least one temperature sensor (26) and/or at least one electrical discharge pole (27) and/or, as at least one stimulation means, at least one optical stimulation diode (15) and/or at least one electromedical stimulation pole (16) and/or at least one electromedical ablation pole (28).

7. Base element (1) according to one of the preceding claims, wherein the base element (1) has a compensation means (17), in particular a compensation circuit, which is designed for temperature compensation during the processing of a sensor signal by the processing module (5).

8. Base element (1) according to one of the preceding claims, wherein the base element (1), in particular the at least one sensor module (4), comprises a detection device (29), in particular a detection circuit, for detecting a tissue condition or tissue contact, in particular wherein the detection device (29) is designed for impedance measurement.

9. Base element (1) according to one of the preceding claims, wherein the carrier element (3) consists of printed circuit board foil, in particular a blank cut from printed circuit board foil.

10. Base element (1) according to one of the preceding claims, wherein the carrier element (3) consists of a flat cut-out of printed circuit board foil, in particular which is unfolded in the position of use of the base element (1) to form a three-dimensional, preferably elongated body, in particular a preferably straight prism or a cylinder.

11. Base element (1) according to the preceding claim, wherein the at least one sensor module (4) and/or the at least one processing module (5) and/or the at least one data line (6) are arranged on one side of the carrier element (3) which forms an inner side (17) of the three-dimensional body.

12. Electromedical device (2), namely implantable electromedical electrode and/or electromedical catheter, having at least one base element (1) according to one of the preceding claims.

13. Electromedical device (2) according to the preceding claim having at least two base elements (1) according to one of the preceding claims, whose processing modules (5) are connected to a common data line (22) of the electromedical device (2), in particular wherein the data line (6, 22) is an electrical and/or optical data line, in particular an optical fiber.

14. Electromedical device (2) according to one of claims 12 or 13, wherein two interconnected base elements (1) at least partially overlap each other.

15. Electromedical device (2) according to one of claims 12 to 14, wherein processing modules (5) of base elements (1) of the electromedical device are part of a data bus system of the electromedical device (2).

16. Electromedical device (2) according to one of claims 12 to 15, having a receiving unit (10), in particular having an evaluation unit (23), to which the at least one base element (1) is at least indirectly connected, and/or having a medical pulse generator (24) to which the at least one base element (1) is at least indirectly connected.

17. Electromedical device (2) according to one of claims 12 to 16, having a sheath (25), in particular having a sheath tube, within which the at least one base element (1) is arranged.

18. Electromedical device (2) according to one of claims 12 to 17, wherein the electromedical device (2) is designed as an implantable electromedical electrode, specifically as a mapping electrode and/or sensing electrode and/or ablation electrode, and/or as an electromedical catheter.

## Revendications

1. Élément de base (1) pour un dispositif électromédical (2), à savoir une électrode électromédicale implantable et/ou un cathéter électromédical, lequel élément de base (1) comporte un élément de support (3) sur lequel sont disposés au moins un module de capteur (4) et/ou au moins un moyen de stimulation (15, 16, 28), au moins un module de traitement de signaux (5) connecté au module de capteur (4) et/ou au moyen de stimulation (15, 16, 28) et au moins une ligne de signaux (6) à laquelle le module de traitement de signaux (5) est connecté.

2. Élément de base (1) selon la revendication précédente, dans lequel le module de traitement de signaux (5) est configuré pour traiter un signal de capteur généré avec le module de capteur (4) sur l'élément de base (1).

3. Élément de base (1) selon l'une des revendications précédentes, dans lequel au moins une, en particulier deux interfaces de raccordement (7) sont prévues sur l'élément de support (3) en vue du raccordement de l'élément de base (1) à un autre élément de base ou un élément de base (1) de construction similaire selon l'une des revendications précédentes, et/ou dans lequel sont prévues sur l'élément de support (3) au moins une, en particulier deux interfaces de connexion (8) pour la connexion de l'élément de base (1) à un autre élément de base ou un élément de base (1) de construction similaire selon l'une des revendications précédentes .

4. Élément de base (1) selon la revendication précédente, dans lequel l'interface de raccordement (7) et/ou l'interface de connexion sont formées par un plot de brasure sur l'élément de support (3).

5. Élément de base (1) selon l'une des revendications précédentes, dans lequel au moins un module de traitement de signaux (5) comprend un circuit intégré (9) spécifique de l'application, qui est configuré pour convertir, en particulier en une force, un signal de capteur généré par le module de capteur (4), en particulier par une ou la jauge extensométrique du module de capteur (4), et/ou pour transmettre la grandeur d'analyse et/ou le signal de capteur via la ligne de signaux (6) à une unité de réception (10) qui peut être connectée au moins indirectement à l'élément de base (1).

6. Élément de base (1) selon l'une des revendications précédentes, dans lequel l'élément de base (1), en particulier l'au moins un module de capteur (4), comporte au moins une jauge extensométrique (11) et/ou au moins un capteur de force (12) et/ou au moins un capteur d'accélération (30) et/ou au moins un capteur biochimique (13) et/ou au moins un biocapteur (14) pour des états biochimiques du corps, en particulier pour la détection d'une inflammation, et/ou au moins une sonde de température (26) et/ou au moins un pôle de dérivation électrique (27) et/ou au moins une diode de stimulation optique (15) constituant l'au moins un moyen de stimulation et/ou au moins un pôle de stimulation électromédical (16) et/ou au moins un pôle d'ablation électromédical (28).

7. Élément de base (1) selon l'une des revendications précédentes, dans lequel l'élément de base (1) comporte un moyen de compensation (17), en particulier un circuit de compensation, configuré en vue d'une compensation de la température lors du traitement d'un signal de capteur par le module de traitement de signaux (5).

8. Élément de base (1) selon l'une des revendications précédentes, dans lequel l'élément de base (1), en particulier l'au moins un module de capteur (4), comporte un dispositif de détection (29), en particulier un circuit de détection, pour la détection d'un état de tissus ou d'un contact avec des tissus, lequel dispositif de détection (29) est en particulier configuré pour mesurer l'impédance.

9. Élément de base (1) selon l'une des revendications précédentes, dans lequel l'élément de support (3) se compose d'un film pour circuits imprimés, en particulier d'une découpe d'un film pour circuits imprimés.

10. Élément de base (1) selon l'une des revendications précédentes, dans lequel l'élément de support (3) se compose d'une découpe plane d'un film de circuits imprimés, qui est en particulier déployée dans la position d'utilisation de l'élément de base (1) pour donner un élément à trois dimensions, de préférence allongé, en particulier un prisme, de préférence rectiligne, ou un cylindre.

11. Élément de base (1) selon la revendication précédente, dans lequel l'au moins un module de capteur (4) et/ou l'au moins un module de traitement de signaux (5) et/ou l'au moins une ligne de signaux (6) sont disposés sur un côté de l'élément de support (3) qui forme une face intérieure (17) de l'élément à trois dimensions.

12. Dispositif électromédical (2), à savoir électrode électromédicale implantable et/ou cathéter électromédical, muni d'au moins un élément de base (1) selon l'une des revendications précédentes.

13. Dispositif électromédical (2) selon la revendication précédente muni d'au moins deux éléments de base (1) selon l'une des revendications précédentes, dont les modules de traitement de signaux (5) sont connectés à une ligne de signaux (22) commune du dispositif électromédical (2), en particulier dans lequel la ligne de signaux (6, 22) est une ligne de signaux électrique et/ou optique, en particulier un guide d'ondes lumineuses.

14. Dispositif électromédical (2) selon l'une des revendications 12 ou 13, dans lequel deux éléments de base (1) connectés l'un à l'autre se chevauchent au moins partiellement.

15. Dispositif électromédical (2) selon l'une des revendications 12 à 14, dans lequel des modules de traitement de signaux (5) d'éléments de base (1) du dispositif électromédical font partie d'un système de bus de signaux du dispositif électromédical (2).

16. Dispositif électromédical (2) selon l'une des revendications 12 à 15 muni d'une unité de réception (10), en particulier d'une unité d'analyse (23), à laquelle l'au moins un élément de base (1) peut être au moins indirectement connecté, et/ou avec un générateur d'impulsions médical (24) auquel l'au moins un élément de base (1) peut être au moins indirectement connecté.

17. Dispositif électromédical (2) selon l'une des revendications 12 à 16 muni d'une enveloppe (25), en particulier d'une gaine d'enveloppe, à l'intérieur de laquelle l'au moins un élément de base (1) est disposé.

18. Dispositif électromédical (2) selon l'une des revendications 12 à 17, dans lequel le dispositif électromédical (2) est conformé comme une électrode électromédicale implantable, à savoir comme une électrode de cartographie et/ou une électrode de détection et/ou une électrode d'ablation, ou comme un cathéter électromédical.
